# EUROPEAN PATENT APPLICATION

(11) **EP 4 725 569 A1**
(43) Date of publication of application: **15.04.2026**
(21) Application number: 25208015.5
(22) Date of filing: 10.10.2025
(51) Int. Cl.: A63F 13/54, A61B 5/12, H04R 5/04, A63F 13/67, A63F 13/22, A63F 13/63

(54) **SYSTEM AND METHOD FOR MODIFYING VIDEO GAME AUDIO**

(30) Priority: 11.10.2024 GB 202415014
(71) Applicant: Sony Interactive Entertainment Inc., Tokyo 108-0075 (JP)
(72) Inventor: ARMSTRONG, Calum, London, W1F 7LP (GB); BUCHANAN, Christopher George, London, W1F 7LP (GB); SCHEMBRI, Danjeli, London, W1F 7LP (GB)
(74) Representative: Fish & Richardson P.C.

(57) **Abstract**

A computer-implemented method for modifying video game audio output using a video gaming system. The method comprises: obtaining a hearing loss profile associated with a user, the hearing loss profile defining a hear loss value varying with frequency; obtaining audio data to be output on a video gaming system, the audio data corresponding to an audio segment spanning a period of time, the audio data comprising a plurality of frequency bands; determining, for each frequency band of the plurality of frequency bands, a respective loudness level; selecting, for each frequency band of the plurality of frequency bands of the audio data, a respective modulation profile based on the frequency band and the respective loudness levels, the modulation profile defining a gain value to be applied dependent on a hearing loss value provided by the hearing loss profile; and applying, to each frequency band of the audio data, the respective modulation profiles using the hearing loss profile to generate modified video game audio.

## Description

### FIELD OF THE INVENTION

The present invention is in the field of video gaming systems and relates to systems and methods for modifying video game audio.

### BACKGROUND

Audio is a key contributor to user enjoyment when playing video games. Types of audio used in video games typically include background music, ambient sound during a video game scene, and voice chat with other users playing the same game. High-quality audio enhances the immersive experience and overall enjoyment of the game.

Many users, however, are affected by hearing loss or impairment, which can significantly impact their ability to hear video game audio and thus diminish their gaming experience.

Some users may have moderate to severe hearing loss. These individuals may use hearing aids, typically obtained via prescription. They often have access to an audiogram, which is a graph showing the user's hearing sensitivity across various frequencies, detailing their hearing profile. Despite the availability of hearing aids, these devices are generally incompatible with gaming systems, posing a challenge for this user group in accessing game audio effectively.

Other users have low to moderate hearing loss, often caused by aging or other factors such as tinnitus. This group is typically less likely to use prescription hearing aids, relying instead on other means to compensate for their hearing impairment.

There is a significant need to assist users affected by hearing loss or impairment in the video gaming context. Traditional hearing aids are designed with the assumption that a substantial portion of environmental audio is unimportant, focusing on amplifying certain key frequencies. In contrast, video game audio is meticulously curated, with each sound element intentionally included to enhance the gaming experience. Therefore, preserving as much of the audio as possible is crucial for maintaining the integrity and enjoyment of the game for users with hearing impairments.

Furthermore, existing hearing aid hardware is not compatible with video gaming systems and as such, their utility is limited in the context of video game audio quality.

There is therefore a need for a method that overcomes these issues and facilitates clearer audio whilst minimizing any loss of audio quality.

### SUMMARY OF INVENTION

In a first aspect of the invention, there is provided a computer-implemented method for modifying video game audio output using a video gaming system, the method comprising: obtaining a hearing loss profile associated with a user, the hearing loss profile defining a hearing loss value varying with frequency; obtaining audio data to be output on a video gaming system, the audio data corresponding to an audio segment spanning a period of time, the audio data comprising a plurality of frequency bands; determining, for each frequency band of the plurality of frequency bands, a respective loudness level; selecting, for each frequency band of the plurality of frequency bands of the audio data, a respective modulation profile based on the frequency band and the respective loudness levels, the modulation profile defining a gain value to be applied dependent on a hearing loss value provided by the hearing loss profile; and applying, to each frequency band of the audio data, the respective modulation profiles using the hearing loss profile to generate modified video game audio.

The "video game audio" may refer to audio data associated with a video game run on the video gaming system. The video game audio data may originate from one or more game files comprising game logic. The "hearing loss profile" may be understood as data that describes how sensitive a user is to different frequencies. The "audio segment" may be understood as a portion of the video game audio that is to be output over the period of time. The "loudness level" may refer to a range of amplitudes or "loudness" that the frequency band falls within. The "modulation profile" may be understood as a gain curve configured to provide gain values to be applied depending on hearing loss levels (e.g., as provided by the hearing loss profile).

In the present invention, a frequency band of video game audio may be modified by a modulation profile determined or selected based on the loudness level of the frequency band. Depending on the loudness level (e.g., a high or low loudness level) of the frequency band, a different modulation profile is determined or selected. The modification of the frequency band is also dependent on the hearing loss profile associated with the user. Accordingly, the present invention may provide a means for modifying video game audio according to a user's hearing loss profile, whilst also taking the loudness levels of frequency bands into account. Advantageously, the present invention may provide modified video game audio that is adapted to the user's unique hearing loss profile for different frequency bands and is dependent on the loudness of the frequency bands, thereby providing clearer audio whilst minimizing any loss of audio quality.

In some embodiments, the modulation profiles are gain curves, defining a gain to be applied dependent on a hearing loss value. In this way, there may be no reduction in amplitude for any frequency band. Advantageously, there may be a minimal loss of audio quality.

Preferably, each modulation profile comprises a non-zero gain value for all non-zero hearing loss values. In this way, all frequency bands are modulated or boosted. As discussed above, in the context of video game audio, the audio is meticulously curated, with each sound element intentionally included to enhance the gaming experience. Since each modulation profile originates at the origin, all frequency bands may be modulated or boosted. Advantageously, audio quality may be improved without sacrificing any audio elements.

In some embodiments, the hearing loss profile is an audiogram. Advantageously, users who have access to an audiogram may use the audiogram, which is tailored to their specific hearing loss profile, thereby providing a greater quality of modified video game audio for that particular user.

In some embodiments, the method further comprises receiving a user selection of a hearing loss profile from a plurality of premade hearing loss profiles. In this way, users having no access to an audiogram may still select a hearing loss profile.

In some embodiments, wherein receiving a user selection of the hearing loss profile comprises: outputting, for each frequency band of the plurality of frequency bands, test audio across a range of amplitudes; receiving a user input indicative of the user's sensitivity at each frequency band; and selecting a hearing loss profile from a plurality of premade hearing loss profiles based on the user input. In this way, the hearing loss profile may be tailored to a specific user even if that user does not have access to an audiogram, thereby providing a greater quality of modified game audio even without an audiogram.

In some embodiments, obtaining the hearing loss profile associated with the user comprises: receiving a user input indicative of the hearing loss profile. In this way, a user can input their own hearing loss profile. Advantageously, increased customisation of hearing loss profiles may be provided.

In some embodiments, obtaining a modulation profile comprises inputting the determined loudness into a modulation function, the modulation function defining a gain value as a function of loudness. The modulation function may be continuous such that a gain value can be provided for any frequency and loudness.

In some embodiments, obtaining a modulation profile comprises selecting a modulation curve, each modulation curve corresponding to a discrete loudness level. In some embodiments, determining, for each frequency band of the plurality of frequency bands, a respective loudness level comprises: obtaining an energy of the audio data within the frequency band; and selecting the loudness level from a plurality of discrete loudness levels based on the energy of the frequency band. In some embodiments, selecting the loudness level based on the energy of the frequency band comprises: identifying a first loudness level from a plurality of predetermined loudness levels, the first loudness level covering the energy of the frequency band; and selecting the first loudness level as the loudness level for the frequency band. Advantageously, the loudness level of the frequency band may be more easily categorized.

In some embodiments, determining the discrete loudness level comprises: identifying a first loudness level from a plurality of predetermined discrete loudness levels; identifying a second loudness level from the plurality of predetermined discrete loudness levels; wherein the energy of the frequency band is between the first loudness level and the second loudness level; and wherein selecting the modulation profile comprises interpolating a first modulation profile associated with the first loudness level and a second modulation profile associated with the second loudness level to obtain an interpolated modulation profile. In this way, when discrete loudness levels are used, an interpolated modulation profile may be obtained when the energy of a frequency band is between ranges covered by separate discrete loudness levels. Advantageously, a more accurate gain value may be obtained.

**In** some embodiments, obtaining the energy of the frequency band comprises: calculating a Root Mean Square (RMS) value of the frequency band. More specifically, the RMS value of the band-limited audio signal is calculated. Since the frequency band comprises positive and negative amplitude values, the RMS value can provide a more meaningful measure of the energy of the frequency band.

In some embodiments, the energy of the frequency band is obtained from the audio data. In this embodiment, the energy of the frequency band may be present in the audio data associated with that frequency band, for example as metadata. Advantageously, this embodiment may require no calculations at runtime, thereby saving computational resources at runtime.

In some embodiments, applying, to each frequency band of the plurality of frequency bands, the respective modulation profiles comprises: obtaining hearing loss values corresponding to a plurality base frequencies of the frequency band; obtaining gain values from the modulation corresponding to the hearing loss values of the base frequencies; and obtaining gain values corresponding to other frequencies of the frequency band by interpolating the gain values associated with neighbouring base frequencies. The base frequencies may be discrete frequency values distributed across the frequency band. The other frequencies may be frequencies in the frequency band other than the base frequencies. **In** this way, gain values may be obtained from the modulation profile for only the base frequencies, and the gain values for the other frequencies may be obtained by interpolation of neighbouring base frequency gain values. Advantageously, computational resources may be saved.

**In** some embodiments, applying, to each frequency band of the plurality of frequency bands, the respective modulation profiles comprises: determining an average hearing loss value for the frequency band; obtaining a gain value from the modulation profile corresponding to the average hearing loss value; and applying the gain value across the whole frequency band. Advantageously, computing resources may be saved because a single gain value is applied across the whole frequency band.

**In** some embodiments, applying, to each frequency band of the plurality of frequency bands, the respective modulation profiles comprises: obtaining, from the hearing loss profile, a hearing loss value for each frequency value of the frequency band; obtaining, for each hearing loss value, a corresponding gain value from the modulation profile; and applying the gain value to the frequency. Advantageously, audio quality may be further improved because each frequency of the frequency band may be boosted according to the user's hearing loss at that frequency.

In some embodiments, the method further comprises: receiving a user selection of the period of time over which the audio segment spans. In this way, the period of time over which the audio segment spans can be modified by the user.

In another aspect of the invention, there is provided a non-transitory storage medium comprising instructions that when executed by a processor cause the processor to perform the method of the first aspect. The processor may comprise a central processing unit (CPU).

In another aspect of the invention, there is provided a computer program comprising instructions that when executed by a processor cause the processor to perform the method of the first aspect. The processor may comprise a central processing unit (CPU).

In another aspect of the invention, there is provided a video gaming system for modifying video game audio, the system comprising a processor and being configured to perform the method of the first aspect.

### BRIEF DESCRIPTION OF DRAWINGS

Figure 1 illustrates an example video gaming system configured to implement methods disclosed herein;
Figure 2 illustrates a method flow diagram of a method according to the present invention;
Figure 3 illustrates an example audiogram showing a user's hearing threshold across different frequencies; and
Figure 4 illustrates a plot of example modulation profiles.

### DETAILED DESCRIPTION

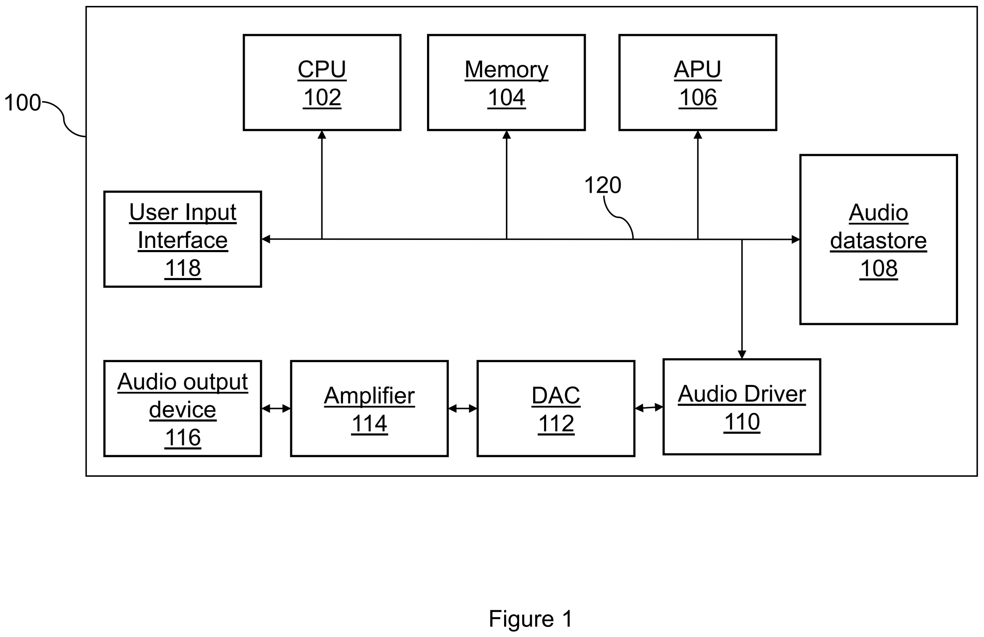

Figure 1 illustrates an example video gaming system 100 configured to implement methods disclosed herein.

The system 100 comprises a central processing unit (CPU) 102; a memory 104; an audio processing unit (APU) 106; an audio datastore 108; audio drivers 110; a digital-to-analogue converter (DAC) 112; an amplifier 114; and an audio output device 116. The system 100 further comprises a system bus 120 arranged to communicatively couple the CPU 102; the memory 104; the APU 106; the audio datastore 108; and the audio driver 110. The system 100 may further comprise other components typical to a video gaming system, such as a user input interface 118 arranged to facilitate communication with a user input device (not shown), such as a video game controller. The system 100 is typically in communication with a network (not shown).

The CPU 102 is arranged to control operation of the system 100. The CPU 102 is in communication with the memory 104 and is arranged to load instructions stored on the memory 104 in order to cause the CPU 102 to perform the methods disclosed herein. The memory 104 may include one or more volatile or non-volatile storage devices, such as random-access memory (RAM), dynamic RAM (DRAM), and/or any other suitable storage medium comprising instructions that when executed by the CPU 102, cause the CPU 102 to perform the method steps disclosed herein. The memory 104 also comprises one or more game files comprising game logic that when executed by the CPU 102, cause the CPU to manage and process audio data.

The APU 106 is arranged to be controlled by the CPU 102. For example, the APU 106 comprises an APU driver (not shown) for controlling operation of the APU 106 to facilitate execution of the methods disclosed herein. The CPU 102 provides audio processing instructions to the APU 106 to process and manage audio data. The APU 106 carries out various tasks, including processing audio signals, managing audio channels, and facilitating high-quality audio output.

The CPU 102 is coupled to the audio datastore 108 such that the CPU 102 can read and write data to the audio datastore 108. The audio datastore 108 may include one or more volatile or non-volatile storage devices, such as random-access memory (RAM), dynamic RAM (DRAM), and/or any other suitable storage medium. The audio datastore 108 may be associated with the memory 104. The audio datastore 108 is arranged to store audio data representative of various audio assets of a particular video game. These audio assets typically comprise but is not limited to comprising sound effect assets; music assets; dialogue assets; and ambient sound assets. Some assets, such as the music assets, may be directly streamed via an input device, such as a disk drive comprising a disk, or the network.

The audio driver 110 is arranged to facilitate communication between software components and hardware components. The audio driver 110 is arranged to provide data transfer and format conversion. The audio driver 110 receives audio data from the APU 106, performs one or more format conversion operations, and provides digital audio signals to the DAC 112.

The DAC 112 is arranged to receive digital audio signals and convert the digital audio signals into analogue audio signals suitable for output. The DAC 112 receives the digital audio signals from the audio driver 110, converts the digital audio signals into analogue audio signals, and outputs the analogue audio signals to the amplifier 114.

The amplifier 114 is arranged to receive the analogue signals from the amplifier 114 and boosts the analogue audio signals to a level suitable for driving the audio output device 116. The amplifier 114 is particularly useful in the present invention which requires different levels of audio boosting for different frequency bands.

The audio output device 116 is arranged to receive the boosted analogue audio signals from the amplifier 114 and generate sound waves based on these boosted analogue audio signals. The audio output device 116 could be any device suitable for generating sound waves. For example, the audio output device 116 could be a speaker 116 or a headset 116.

It will be understood that the system 100 is not limited to these components and typically includes other features, such as a GPU (not shown) arranged to be controlled by the CPU 102. For example, the CPU 102 comprises a GPU driver (not shown) for controlling operation of the GPU to facilitate execution of various graphics processing operations. Typically, the CPU 102 provides graphics rendering instructions to the GPU. To process the graphics rendering instructions, the CPU 102 controls the GPU to perform the rendering of graphics data. These graphics rendering instructions conform to a graphics application programming interface (API) such that the GPU can efficiently implement graphics rendering steps. The GPU comprises a GPU memory (not shown) arranged to store visual data such as textures, geometry, and other graphical assets. The GPU memory includes, for example, video random access memory (VRAM). The GPU may additionally carry out some audio processing tasks, for example for spatial audio in 3D environments.

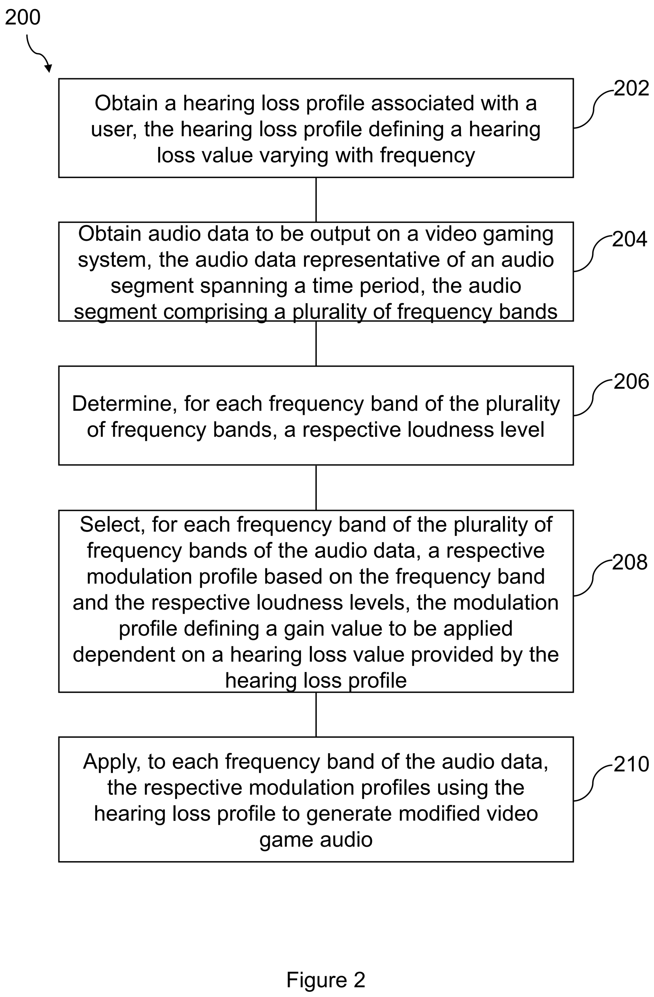

Figure 2 illustrates a method flow diagram of a method according to the present invention. More particularly, Figure 2 illustrates a computer implemented method 200 for modifying video game audio using the video gaming system 100. One or more steps of the method 200 are implemented by the CPU 102 and other components of the system 100.

Step 202 comprises obtaining a hearing loss profile associated with a user, the hearing loss profile defining a hearing loss value varying with frequency.

The hearing loss profile is a representation of the user's hearing ability at different frequencies. More particularly, the hearing loss profile describes the user's hearing sensitivity at various frequencies or frequency bands.

**In** some implementations, the hearing loss profile is an audiogram. The audiogram is represented as data through a set of numerical values that correspond to hearing thresholds at various frequencies for both ears. The hearing thresholds correspond to the lowest intensity of sounds that the user can hear across a range of frequencies. Respective hearing thresholds are associated with the left ear and the right ear. Using a 125 Hz frequency as an example, a first hearing threshold associated with the user's left ear may be 20 dB, whilst a second hearing threshold associated with the user's right ear may be 25 dB. This implementation is useful for users who have access to an audiogram.

Obtaining the audiogram associated with the user could comprise: accessing a memory (e.g., the memory 104) and extracting the audiogram data. The user could upload an audiogram file (for example, a CSV file) to the video gaming system 100 (i.e., the memory 104) and subsequently interact with the UI to select the audiogram.

**In** an alternative implementation, the hearing loss profile is a hearing loss profile selected from a plurality of premade or preset hearing loss profiles. The plurality of premade hearing loss profiles are predetermined according to a range of different hearing loss profile types. For example, a first premade hearing loss profile may comprise hearing thresholds that are higher at higher frequencies, and lower at lower frequencies. This first premade hearing loss profile may be particularly suitable for users with tinnitus, because higher frequency sounds may be obscured or masked by the tinnitus, thereby negatively affecting the user's ability to hear these higher frequency sounds. A second premade hearing loss profile may comprise a hearing threshold that is higher at lower frequencies and higher at higher frequencies. It will be appreciated that these first and second premade hearing loss profiles are by way of example only, and there may be many more premade hearing loss profiles. This implementation is useful for users who may not have access to an audiogram.

In this alternative implementation, the step of obtaining 202 the hearing loss profile associated with the user could comprise: receiving a user selection of a hearing loss profile from a plurality of premade hearing loss profiles. Receiving a user selection of the hearing loss profile comprises: outputting, via the audio output device 116, for each frequency band of a plurality of frequency bands, test audio across a range of amplitudes; receiving a user input indicative of the user's sensitivity at each frequency band; and selecting a hearing loss profile from a plurality of premade hearing loss profiles based on the user input. The plurality of amplitudes could be discrete or continuous. The video gaming system 100 instructs the user to provide the user input to the video game controller when the user detects (i.e., hears) the output sound. Based on these user inputs, the video gaming system can select a premade hearing loss profile that is the closest match to the user's hearing loss profile.

For example, for the test audio of a first frequency band (e.g., 20 Hz to 250 Hz), the system 100 gradually increases the amplitude until the user provides the user input (i.e., when they hear the test audio). This user input is indicative of the user's sensitivity at this first frequency band.

In a further alternative implementation, the hearing loss profile is input by the user. In this implementation, the step of obtaining 202 the hearing loss profile associated with the user comprises: receiving a user input indicative of the hearing loss profile. For example, the video gaming system 100 may provide an interface for the user to input their hearing loss threshold across different frequencies.

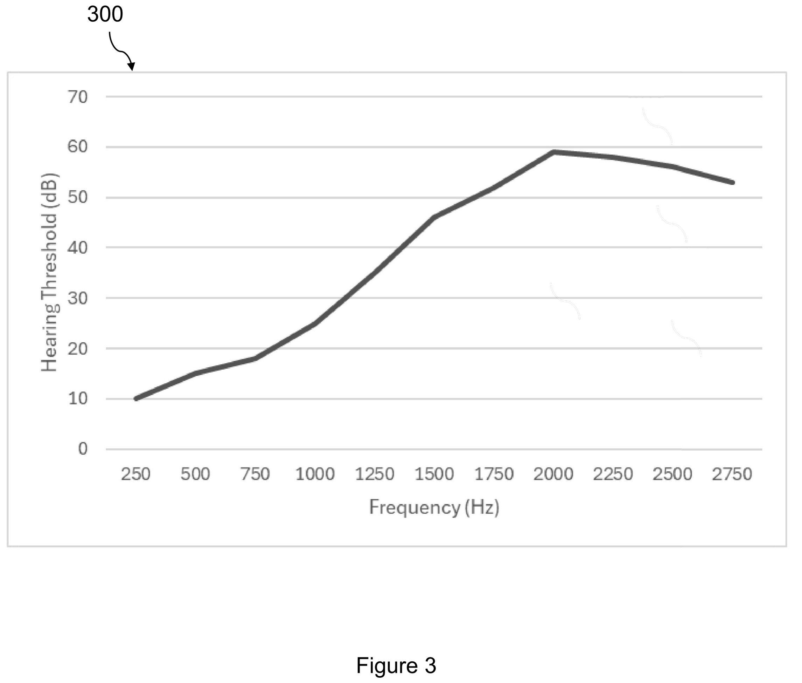

Figure 3 illustrates an example hearing loss profile 300 showing a user's hearing loss across different frequencies. It will be appreciated that the hearing loss profile is simplified and depicts the user's hearing threshold for only a single ear. The method 200 is applied using a hearing loss profile for each ear for a stereo audio output. In some implementations, for example for mono audio playback or loudspeaker playback, the hearing loss profile for both ears is averaged to produce the hearing loss profile used in the present method.

Step 204 comprises obtaining audio data to be output on a video gaming system, the audio data corresponding to an audio segment spanning a period of time, the audio segment comprising a plurality of frequency bands. The processor 102 obtains 204 the audio data from the audio datastore 108.

The "audio segment" corresponds to output audio that is to be output over the period of time. The period of time is not limited and could be over a shorter period or over a longer period. The period of time could be the length of a scene of the video game, although this is not limited. The "scene" is a specific segment of the video game and may be considered a cohesive unit within the games larger structure, for example because a continuous audio asset is provided. The scene can be defined by scene audio. The scene audio comprises different audio elements, including, but not limited to, a background music element, sound effect elements, dialogue elements, and ambient sound elements. These audio segments therefore comprise different audio elements. These audio elements comprise data representative of the plurality of frequency bands. The amplitude or energy of each of the frequency bands can vary over time. The length of the period of time could be pre-programmed. Alternatively, the user could select the period of time such that the method further comprises: receiving a user selection of the period of time over which the audio segment spans.

The range of frequencies used in video game audio typically spans from 20 Hz to 20 kHz. There may be any number of frequency bands. In an example implementation, there are 6 frequency bands spanning the range of frequencies of a particular game. Each frequency band could vary in size such that one frequency band covers a greater range of frequency values than another frequency band. For example, a first frequency band could cover a range of low frequencies from 20 Hz to 250 Hz and a second frequency band could cover a range of high frequencies from 4 kHz to 20 kHz. Alternatively, each frequency band could be of equal size such that one frequency band covers a range of frequencies equal in size to another frequency band.

In a simplified example, there is a first frequency band and a second frequency band. The first frequency band (herein the "low" frequency band) comprises frequencies less than 4 kHz. The second frequency band (herein the "high" frequency band) comprises frequencies greater than or equal to 4 kHz and less than 8 kHz. It will be understood that these frequency bands are for illustration purposes only, and there would typically be a greater number of frequency bands spanning different ranges of frequencies.

Step 206 comprising determining, for each frequency band of the plurality of frequency bands, a respective loudness level. The "loudness level" describes an energy or "loudness" of the frequency band of the audio segment over the period of time.

Determining, for each frequency band of the plurality of frequency bands, a respective loudness level, could comprise: obtaining an energy of the audio data within the frequency band; and selecting the loudness level from a plurality of discrete loudness levels based on the energy of the frequency band. Each loudness level covers a respective range of energies. The "energy" may be understood as a representation of the "loudness" level of a frequency band over the period of time, as discussed further below.

In an example, the plurality of predetermined loudness levels comprises a first loudness level and a second loudness level. The first loudness level represents a first range of energy values, and the second loudness level represents a second range of energy values. The second range of energies spans a range of energy values having a greater magnitude than the range of energy values of the first range of energies. Thus, the first loudness level represents a "quiet" loudness level, and the second loudness level represents a "loud" loudness level. It will be understood that there may be any number of loudness levels, each representing a respective range of energy values.

In some implementations, determining 206, for each frequency band of the plurality of frequency bands, the respective loudness level comprises determining, for each frequency band of the plurality of frequency bands, an energy of the frequency band and selecting the loudness level based on the energy of the frequency band. This implementation typically occurs at runtime.

In one implementation, to obtain the energy of a particular frequency band, the CPU 102 calculates a Root Mean Square (RMS) value of the frequency band over a period of time. In this implementation, for a particular frequency band, the audio data may comprise a series of discrete amplitude values distributed over the period of time. The CPU 102 calculates the RMS value of the series of discrete magnitude values of the audio data for the frequency band. Thus, in this implementation, determining, for each frequency band of the plurality of frequency bands, the energy of the frequency band comprises: calculating an RMS value of the magnitude values of the frequency band. Calculating the RMS value of the magnitude values provides an average energy over the period of time.

Continuing with this implementation, to select the loudness level based on the energy of the frequency band, the CPU 102 identifies which of the plurality of predetermined loudness levels covers the energy of the frequency band. Thus, in this implementation, selecting, for each frequency band of the plurality of frequency bands, the loudness level based on the energy of the frequency band comprises: identifying which of the plurality of predetermined loudness levels covers the energy of the frequency band.

In an alternative implementation, the audio data comprises data that is representative of the energy of a particular frequency band. In this implementation, the energy of a particular frequency band is predetermined and included as part of the game data. Thus, the energy of the frequency band is obtained from the audio data. In this implementation, the energy of a particular frequency band may also have been determined by calculating an RMS value of the frequency band over the period of time. In an alternative implementation, the energy of a particular frequency band could be assigned based on a user-assigned value. For example, a video game designer may manually assess the loudness of a particular frequency band and assign an appropriate loudness level.

Continuing with the example, the loudness level of the low frequency band is determined 206 to be the first loudness level (herein the "loud" loudness level), for example because the energy of the low frequency band is determined to fall within the range of the first loudness level. The loudness level of the high frequency band is determined to be a second loudness level (herein the "quiet" loudness level), for example because the energy of the high frequency band is determined to fall within the range of the second loudness level.

Step 208 comprises selecting, for each frequency band of the plurality of frequency bands of the audio data, a respective modulation profile based on the frequency band and the respective loudness levels, the modulation profile defining a gain value to be applied dependent on a hearing loss value provided by the hearing loss profile.

The modulation profile is a gain curve configured to provide a gain value to be applied for a particular hearing loss level. Each modulation profile comprises a non-zero gain value for all non-zero hearing loss values. For example, each gain curve originates at the origin. Therefore, every frequency is boosted.

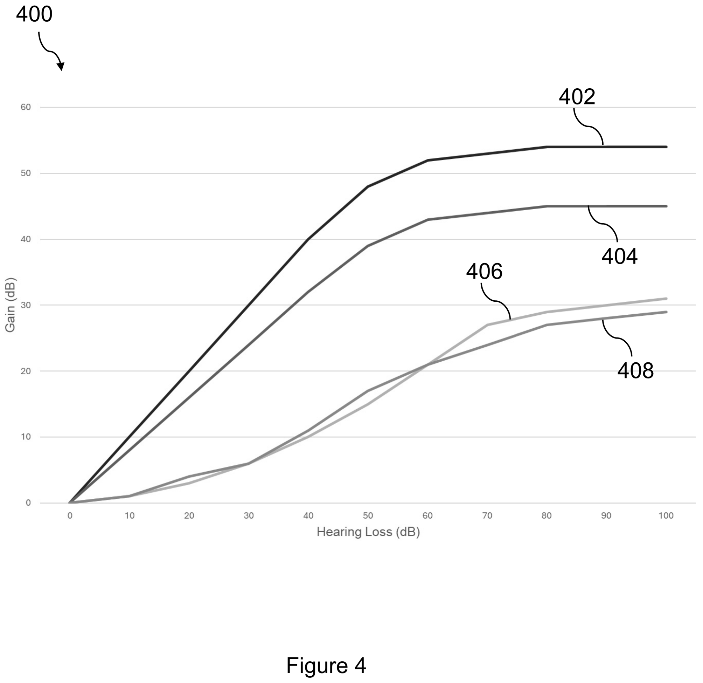

Figure 4 shows a plot 400 of example modulation profiles. The modulation profiles comprise a first modulation profile 402; a second modulation profile 404; a third modulation profile 406; and a fourth modulation profile 408. The first modulation profile 402 is for a first frequency band (e.g., a low frequency band) at a first loudness level (e.g., a "quiet" loudness level). The second modulation profile 404 is for a second frequency band (e.g., a high frequency band) at the quiet loudness level. The third modulation profile 406 is for the low frequency band at a second loudness level (e.g., a "loud" loudness level). The fourth modulation profile 408 is for the high frequency band at the loud loudness level. Each modulation profile originates at the origin so that every frequency is boosted.

The modulation profiles are selected 208 for each frequency band based on the loudness level determined for the frequency band in step 206.

Continuing with the example, in step 206 the loudness level of the low frequency band was determined to be the loud loudness level and the loudness level of the high frequency band was determined to be the quiet loudness level. Therefore, the third modulation profile 406 is selected 208 for the low frequency band and the second modulation profile 404 is selected for the high frequency band.

Step 210 comprises applying, to each frequency band of audio data, the respective modulation profiles using the hearing loss profile to generate modified video game audio.

The hearing loss profile is used to determine the hearing loss value for the frequency band and the hearing loss value is used to determine a corresponding gain value from the modulation profile.

In a first implementation, applying, to each frequency band of the plurality of frequency bands, the respective modulation profiles comprises: obtaining hearing loss values corresponding to a plurality base frequencies of the frequency band; obtaining gain values from the modulation corresponding to the hearing loss values of the base frequencies; and obtaining gain values corresponding to other frequencies of the frequency band by interpolating the gain values associated with neighbouring base frequencies. The base frequencies or anchor frequencies are discrete frequency values distributed across the frequency band. These base frequencies are typically equally distributed across the frequency band. For example, if a frequency band spans the range of 50 Hz to 500 Hz, and there are 6 base frequencies, the base frequencies could be 50 Hz, 140 Hz, 230 Hz, 320 Hz, 410 Hz, and 500 Hz. The other frequencies are frequencies in the frequency band other than the base frequencies. The gain values corresponding to the base frequencies can be obtained by determined the gain value from the modulation profile corresponding to the loudness level of the frequency band. The gain values corresponding to the other frequencies of the frequency band are obtained by interpolating neighbouring gain values. For example, to obtain the gain value for a frequency of 95 Hz, the gain values associated with the base frequencies of 50 Hz and 140 Hz are interpolated.

In an alternative implementation, an average hearing loss value is determined for the frequency band and a corresponding gain value for the average hearing loss value is applied across the whole frequency band. In a simplified example, if a first frequency band comprises hearing loss values of 25 dB, 30 dB, and 35 dB, the average hearing loss value would be 30 dB. The gain value corresponding to the average hearing loss value is applied across the whole frequency band. Using the low frequency band as an example, the third modulation profile 406 was selected in step 208 and so a gain of around 5 dB would be applied if the average hearing loss value was 30 dB. Using the high frequency band as an example, the second modulation profile 404 was selected in step 208 and so a gain of around 25 dB would be applied if the average hearing loss value was 30 dB. This implementation is computationally inexpensive because a single gain value is obtained and applied across the frequency band. It will be appreciated that this gain value is an approximate gain value because a single gain value is applied across the frequency band.

In a further alternative implementation, a hearing loss value for each frequency value of the frequency band is obtained from the hearing loss profile, and a corresponding gain value for the frequency value is applied to the frequency value. This implementation is more computationally expensive than the first implementation because a gain value is obtained for each frequency value of the frequency band. However, this implementation provides a higher quality output.

After applying 210 the respective modulations profiles to each frequency band, modified video game audio is generated. All frequency bands of the initial video game audio have been boosted because each modulation profile originates at the origin. Since each modulation profile provides a different gain value depending on the hearing loss value for different frequency bands or frequencies, and the loudness level of the frequency bands, each frequency band or frequency is boosted by a different amount.

In the present example, the loudness level of the low frequency band is the loud loudness level so the third modulation profile 406 is used. The loudness level of the high frequency band is the quiet loudness level so the second modulation profile 404 is selected for the high frequency band. As shown in Figure 4, the second modulation profile 404 provides a greater boost than the third hearing loss profile 406 and so the low frequency band is boosted more than the high frequency band.

The method 200 provides modified video game audio in which all sounds are boosted to some degree, the amount by which certain frequencies are boosted depending on the hearing loss profile of the user. The modified video game audio sounds clearer and to the user and less audio information is lost when compared to the unmodified video game audio.

In some implementations, determining, for each frequency band of the plurality of frequency bands, a respective loudness level comprises: identifying a first loudness level from a plurality of predetermined discrete loudness levels; identifying a second loudness level from the plurality of predetermined discrete loudness levels; wherein the energy of the frequency band is between the first loudness level and the second loudness level; selecting a respective modulation profile comprises interpolating a first modulation profile associated with the first loudness level and a second modulation profile associated with the second loudness level to obtain an interpolated modulation profile. The interpolated modulation profile comprises a gain curve generated based on the interpolated first and second modulation profiles. Thus, if the energy of the frequency band is outside of a range associated with the first loudness level and outside of a range associated with the second loudness level, the first loudness level and the second loudness level are interpolated. In an example, the first loudness level is associated with a "quiet" modulation curve dictating a compensation gain of 10 for a certain frequency, and the second loudness level is associated with a "loud" modulation curve dictating a compensation gain of 20 for that frequency. For a "medium" energy sound, the modulation curves are interpolated and a compensation gain of 15 is associated with that frequency.

It will be appreciated that the method 200 is applied to audio data corresponding to an audio segment spanning a period of time obtained in step 204. Thus, the method 200 is typically repeated for a plurality of audio segments, such that the modified video game audio is re-calculated for each audio segments. Each audio segment typically comprises different frequency bands having different loudness levels. In some cases, there may be a transient change between a loudness level of a frequency band from a first audio segment to a second audio segment. To reduce sudden increases in gain, historical loudness data may be used to smooth the determination of the loudness level (step 206).

### EMBODIMENTS

Although the present invention is defined in the claims, it should be understood that the present invention can also (alternatively) be defined in accordance with the following embodiments:
1. A computer-implemented method for modifying video game audio output using a video gaming system, the method comprising:
   obtaining a hearing loss profile associated with a user, the hearing loss profile defining a hearing loss value varying with frequency;
   obtaining audio data to be output on a video gaming system, the audio data corresponding to an audio segment spanning a period of time, the audio data comprising a plurality of frequency bands;
   determining, for each frequency band of the plurality of frequency bands, a respective loudness level;
   selecting, for each frequency band of the plurality of frequency bands of the audio data, a respective modulation profile based on the frequency band and the respective loudness levels, the modulation profile defining a gain value to be applied dependent on a hearing loss value provided by the hearing loss profile; and
   applying, to each frequency band of the audio data, the respective modulation profiles using the hearing loss profile to generate modified video game audio.
2. The computer-implemented method of embodiment 1, wherein each modulation profile comprises a non-zero gain value for all non-zero hearing loss values.
3. The computer-implemented method of any preceding embodiment, wherein the hearing loss profile is an audiogram.
4. The computer-implemented method of any of embodiments 1 to 3, further comprising receiving a user selection of a hearing loss profile from a plurality of premade hearing loss profiles.
5. The computer-implemented method of embodiment 4, wherein receiving a user selection of the hearing loss profile comprises:
   outputting, for each frequency band of the plurality of frequency bands, test audio across a range of amplitudes;
   receiving a user input indicative of the user's sensitivity at each frequency band; and
   selecting a hearing loss profile from the plurality of premade hearing loss profiles based on the user input.
6. The computer-implemented method of any of embodiments 1 to 3, wherein obtaining the hearing loss profile associated with the user comprises:
   receiving a user input indicative of the hearing loss profile.
7. The computer-implemented method of any preceding embodiment, wherein obtaining a modulation profile comprises inputting the determined loudness into a modulation function, the modulation function defining a gain value as a function of loudness.
8. The computer-implemented method of any of embodiments 1 to 6, wherein obtaining a modulation profile comprises selecting a modulation curve, each modulation curve corresponding to a discrete loudness level.
9. The computer-implemented method of embodiment 8, wherein determining the discrete loudness level comprises:
   obtaining an energy of the audio data within the frequency band; and
   selecting the loudness level from a plurality of discrete loudness levels based on the energy of the frequency band.
10. The computer-implemented method of embodiment 9, wherein selecting the loudness level based on the energy of the frequency band comprises:
   identifying a first loudness level from the plurality of predetermined loudness levels, the first loudness level covering the energy of the frequency band; and
   selecting the first loudness level as the loudness level for the frequency band.
11. The computer-implemented method of embodiment 8, wherein determining the discrete loudness level comprises:
   identifying a first loudness level from a plurality of predetermined discrete loudness levels;
   identifying a second loudness level from the plurality of predetermined discrete loudness levels;
      wherein the energy of the frequency band is between the first loudness level and the second loudness level; and
   wherein selecting the modulation profile comprises interpolating a first modulation profile associated with the first loudness level and a second modulation profile associated with the second loudness level to obtain an interpolated modulation profile.
12. The computer-implemented method of any of embodiments 9 to 11 , wherein obtaining the energy of the frequency band comprises:
   calculating a Root Mean Square value of the frequency band.
13. The computer-implemented method of any of embodiments 9 to 11, wherein the energy of the frequency band is obtained from the audio data.
14. The method of any preceding embodiment, wherein applying, to each frequency band of the plurality of frequency bands, the respective modulation profiles comprises:
   obtaining hearing loss values corresponding to a plurality of base frequencies of the frequency band;
   obtaining gain values from the modulation corresponding to the hearing loss values of the base frequencies; and
   obtaining gain values corresponding to other frequencies of the frequency band by interpolating the gain values associated with neighbouring base frequencies.
15. The computer-implemented method of any preceding embodiment, wherein applying, to each frequency band of the plurality of frequency bands, the respective modulation profiles comprises:
   determining an average hearing loss value for the frequency band;
   obtaining a gain value from the modulation profile corresponding to the average hearing loss value; and
   applying the gain value across the whole frequency band.
16. The computer-implemented method of any preceding embodiment, wherein applying, to each frequency band of the plurality of frequency bands, the respective modulation profiles comprises:
   obtaining, from the hearing loss profile, a hearing loss value for each frequency value of the frequency band;
   obtaining, for each hearing loss value, a corresponding gain value from the modulation profile; and
   applying the gain value to the frequency.
17. The computer-implemented method of any preceding embodiment, further comprising:
   receiving a user selection of the period of time over which the audio segment spans.
18. A non-transitory storage medium comprising instructions that when executed by a processor cause the processor to perform the method of any preceding embodiment.
19. A computer program comprising instructions that when executed by a processor cause the processor to perform the method of any of embodiments 1 to 17.
20. A video game system for modifying audio data, the system comprising a processor and being configured to perform the method of any of embodiments 1 to 17.

## Claims

1. A computer-implemented method for modifying video game audio output using a video gaming system, the method comprising:
obtaining (202) a hearing loss profile associated with a user, the hearing loss profile defining a hearing loss value varying with frequency;
obtaining (204) audio data to be output on a video gaming system, the audio data corresponding to an audio segment spanning a period of time, the audio data comprising a plurality of frequency bands;
determining (206), for each frequency band of the plurality of frequency bands, a respective loudness level;
selecting (208), for each frequency band of the plurality of frequency bands of the audio data, a respective modulation profile based on the frequency band and the respective loudness levels, the modulation profile defining a gain value to be applied dependent on a hearing loss value provided by the hearing loss profile; and
applying (210), to each frequency band of the audio data, the respective modulation profiles using the hearing loss profile to generate modified video game audio.

2. The computer-implemented method of claim 1, wherein each modulation profile comprises a non-zero gain value for all non-zero hearing loss values.

3. The computer-implemented method of any one of claims 1 or 2, wherein the hearing loss profile is an audiogram.

4. The computer-implemented method of any one of claims 1 to 3, further comprising receiving a user selection of a hearing loss profile from a plurality of premade hearing loss profiles, optionally wherein receiving a user selection of the hearing loss profile comprises
outputting, for each frequency band of the plurality of frequency bands, test audio across a range of amplitudes,
receiving a user input indicative of the user's sensitivity at each frequency band, and
selecting a hearing loss profile from the plurality of premade hearing loss profiles based on the user input; and/or
wherein obtaining the hearing loss profile associated with the user comprises:
receiving a user input indicative of the hearing loss profile.

5. The computer-implemented method of any one of claims 1 to 4, wherein obtaining a modulation profile comprises inputting the determined loudness into a modulation function, the modulation function defining a gain value as a function of loudness.

6. The computer-implemented method of any one of claims 1 to 4, wherein obtaining a modulation profile comprises selecting a modulation curve, each modulation curve corresponding to a discrete loudness level.

7. The computer-implemented method of claim 6, wherein determining the discrete loudness level comprises:
obtaining an energy of the audio data within the frequency band; and
selecting the loudness level from a plurality of discrete loudness levels based on the energy of the frequency band; optionally
wherein selecting the loudness level based on the energy of the frequency band comprises:
identifying a first loudness level from the plurality of predetermined loudness levels, the first loudness level covering the energy of the frequency band; and
selecting the first loudness level as the loudness level for the frequency band.

8. The computer-implemented method of claim 6, wherein determining the discrete loudness level comprises:
identifying a first loudness level from a plurality of predetermined discrete loudness levels;
identifying a second loudness level from the plurality of predetermined discrete loudness levels;
wherein the energy of the frequency band is between the first loudness level and the second loudness level; and
wherein selecting the modulation profile comprises interpolating a first modulation profile associated with the first loudness level and a second modulation profile associated with the second loudness level to obtain an interpolated modulation profile.

9. The computer-implemented method of any one of claims 7 or 8, wherein obtaining the energy of the frequency band comprises calculating a Root Mean Square value of the frequency band; and/or
wherein the energy of the frequency band is obtained from the audio data.

10. The computer-implemented method of any one of claims 1 to 9, wherein applying, to each frequency band of the plurality of frequency bands, the respective modulation profiles comprises:
obtaining hearing loss values corresponding to a plurality of base frequencies of the frequency band;
obtaining gain values from the modulation corresponding to the hearing loss values of the base frequencies; and
obtaining gain values corresponding to other frequencies of the frequency band by interpolating the gain values associated with neighbouring base frequencies.

11. The computer-implemented method of any one of claims 1 to 10, wherein applying, to each frequency band of the plurality of frequency bands, the respective modulation profiles comprises:
determining an average hearing loss value for the frequency band;
obtaining a gain value from the modulation profile corresponding to the average hearing loss value; and
applying the gain value across the whole frequency band.

12. The computer-implemented method of any one of claims 1 to 11, wherein applying, to each frequency band of the plurality of frequency bands, the respective modulation profiles comprises:
obtaining, from the hearing loss profile, a hearing loss value for each frequency value of the frequency band;
obtaining, for each hearing loss value, a corresponding gain value from the modulation profile; and
applying the gain value to the frequency.

13. The computer-implemented method of any one of claims 1 to 12, further comprising:
receiving a user selection of the period of time over which the audio segment spans.

14. A computer program comprising instructions that, when executed by a processor (102), cause the processor to perform the method of any one of claims 1 to 13.

15. A video game system (100) for modifying audio data, the system comprising a processor (102) and being configured to perform the method of any one of claims 1 to 13.
